# EUROPEAN PATENT APPLICATION

(11) **EP 4 321 512 A1**
(43) Date of publication of application: **14.02.2024**
(21) Application number: 22799150.2
(22) Date of filing: 06.05.2022
(51) Int. Cl.: C07D 403/06, A61K 31/5377, A61P 3/04, A61P 3/10, A61P 29/00, A61P 15/10

(54) **CO-CRYSTAL OF MELANOCORTIN RECEPTOR AGONIST COMPOUND AND VANILLIN AND METHOD FOR PREPARING SAME**

(30) Priority: 07.05.2021 KR 20210059376
(71) Applicant: LG Chem, Ltd., Seoul 07336 (KR)
(72) Inventor: KIM, Ji Yoon, Daejeon 34122 (KR); CHUN, Seul Ah, Daejeon 34122 (KR); KIM, Sung Won, Daejeon 34122 (KR)
(74) Representative: Hoffmann Eitle
(86) International application number: PCT/KR2022/006482
(87) International publication number: WO 2022/235107

(57) **Abstract**

The present invention relates to a co-crystal of a compound represented by chemical formula 1 and vanillin, a method for preparing same, and a pharmaceutical composition comprising same. The co-crystal of the compound represented by chemical formula 1 and vanillin has excellent chemical and physical stability and is characterized by the XRPD pattern, DSC profile, and/or TGA profile.

## Description

### TECHNICAL FIELD

### [Cross-reference to Related Application]

The present invention claims the benefit of priority based on Korean Patent Application No. 10-2021-0059376, filed on May 7, 2021, the entire disclosure of which is incorporated as part of the specification.

### [Technical Field]

The present invention relates to a co-crystal of a novel compound exhibiting an excellent agonistic activity for a melanocortin receptor, a method for preparing the same, and a pharmaceutical composition comprising the same.

### BACKGROUND ART

Leptin protein is a hormone secreted by adipocytes, and its secretion amount increases with an increase in body fat content. It regulates functions of various neuropeptides produced from hypothalamus, thereby regulating various in vivo functions, including appetite, body fat content, and energy metabolism (Schwartz, et al., Nature 404, 661-671 (2000)). The leptin protein signal transduction for controlling appetite and body weight is made through the regulation of many factors downstream, the most representative of which are melanocortin, agouti-related peptide (AgRP), and neuropeptide Y (NPY) hormones.

When the concentration of leptin in the blood increases as a result of excess calories in vivo, the secretion of proopiomelanocortin (POMC) protein hormone from the pituitary gland increases and the production of AgRP and NPY decreases. A small peptide hormone, alpha-melanocyte-stimulating hormone (MSH), is produced from POMC neurons. The hormone is an agonist for melanocortin-4 receptors (MC4R) of second-order neurons and ultimately induces appetite decrease. Meanwhile, when the concentration of leptin decreases as a result of calorie deficiency, the expression of AgRP, an MC4R antagonist, increases, and the expression of NPY also increases, which ultimately promotes appetite. That is, according to the change of leptin, the alpha-MSH hormone and the AgRP hormone act as agonists and antagonists for MC4R and thus are involved in appetite control.

The Alpha-MSH hormone binds to three MCR subtypes in addition to MC4R to induce various physiological reactions. Five MCR subtypes have been identified so far. Among them, MC1R is expressed mainly in skin cells and is involved in regulating melanin pigmentation (skin pigmentation). MC2R is expressed mainly in the adrenal gland and is known to be involved in the production of glucocorticoid hormones. Its ligand is only adrenocorticotropic hormone (ACTH) derived from POMC. MC3R and MC4R, which are expressed mainly in the central nervous system, are involved in regulating appetite, energy metabolism, and body fat storage efficiency, and MC5R expressed in various tissues is known to regulate exocrine function (Wikberg, et al., Pharm Res 42 (5) 393-420 (2000)). In particular, activation of the MC4R receptor induce appetite decrease and energy metabolism increase and thus has an effect of efficiently reducing body weight. Therefore, it has been proven to be a major action point in the development of anti-obesity drugs (Review: Wikberg, Eur. J. Pharmacol 375, 295-310 (1999)); Wikberg, et al., Pharm Res 42 (5) 393-420 (2000) ; Douglas et al., Eur J Pharm 450, 93-109 (2002); O'Rahilly et al., Nature Med 10, 351-352 (2004)).

The role of MC4R in the control of appetite and body weight was primarily demonstrated through an experiment in an animal model of abnormal expression of the agouti protein (agouti mouse). In the case of the Agouti mouse, it was found that due to genetic mutation, the agouti protein was expressed at a high concentration in the central nervous system and acted as an antagonist of MC4R in the hypothalamus to cause obesity (Yen, TT et al., FASEB J. 8, 479-488 (1994); Lu D., et al. Nature 371, 799-802 (1994)). Subsequent research results showed that the agouti-related peptides (AgRP) similar to the actual agouti protein were expressed in hypothalamic nerves, and these are also known to be antagonists for MC4R and be involved in controlling appetite (Shutter, et al., Genes Dev., 11, 593-602 (1997); Ollman, et al. Science 278, 135-138 (1997)).

Intracerebral administration of alpha-MSH, which is an in vivo MC4R agonist, to animals leads to the effect of reducing appetite. When treating the animals with SHU9119 (peptide) or HS014 (peptide), which are MC4R antagonists, it was observed that appetite increased again (Kask et al., Biochem. Biophys. Res. Comm. 245, 90-93 (1998)). In addition, in animal studies using Melanotan II (MTII, Ac-Nle-c[Asp-His-DPhe-Arg-Trp-Lys]-NH2) and the similar agonist thereof, HP228, after intracerebral, intraperitoneal, or subcutaneous administration, efficiencies of inhibiting appetite, reducing body weight, increasing energy metabolism, etc. were found. (Thiele T. E., et al. Am J Physiol 274 (1 Pt 2), R248-54 (1998); Lee M. D., et al. FASEB J 12, A552 (1998); Murphy B., et al. J Appl Physiol 89, 273-82 (2000)). On the contrary, administration of the representative SHU9119 to animals showed significant and sustained feed intake and weight gain, providing pharmacological evidence that MCR agonists could be anti-obesity agents. The effect of reducing appetite, which is clearly exhibited upon administration of MTII, was not observed in MC4R knock-out (KO) mice. This experimental result proves again that the appetite-reducing effect is achieved mainly through the activation of MC4R (Marsh, et al., Nat Genet 21, 119-122 (1999)).

Anorectic agents acting on the central nervous system were the main types of anti-obesity drugs developed so far. Among them, most were drugs that modulate the action of neurotransmitters. Examples include noradrenalin agents (phentermine and mazindol), serotonergic agents, fluoxetine and sibutramine, and the like. However, the neurotransmitter modulators have a wide range of effects on various physiological actions in addition to appetite suppression, through numerous subtype receptors. Accordingly, the modulators lack selectivity for each subtype, and thus have a major disadvantage in that they are accompanied by various side effects when administered for a long period.

Meanwhile, melanocortin agonists are neuropeptides, not neurotransmitters. Given that in MC4R gene KO mice, all functions other than energy metabolism are normal, they have an advantage as an action point in that they can induce only weight loss through appetite suppression without affecting other physiological functions. In particular, the receptor is a G-protein coupled receptor (GPCR) that belongs to the most successful category of new drug action points developed so far. Thus, the action point greatly differ from existing action points in that it is relatively easy to secure selectivity for subtype receptors.

As an example of utilizing a melanocortin receptor as an action point, international publication nos. WO 2008/007930 and WO 2010/056022 disclose compounds as agonists of the melanocortin receptor.

In addition, the inventors of the present invention have conducted extensive studies and invented a novel compound of the following formula 1 having an excellent agonistic activity selective for a melanocortin receptor, in particular, melanocortin-4 receptor (MC4R), and a method for preparing the same (application no. KR 10-2019-0141649 (filed on 7 November 2019)): 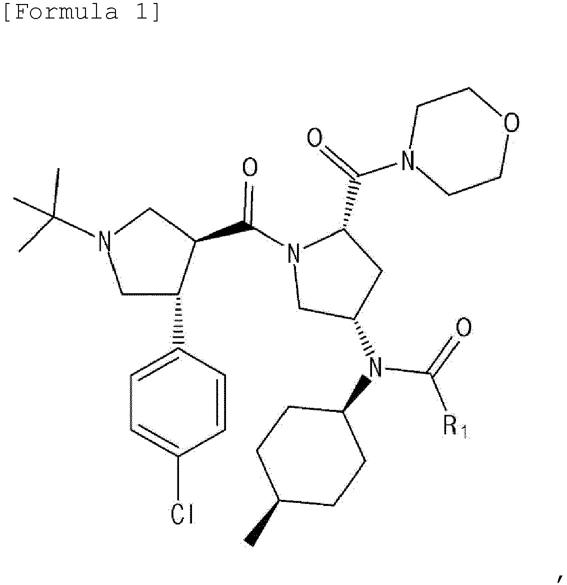 wherein R₁ is a C₂-C₅ alkyl.

Meanwhile, the crystal structure of a pharmaceutically active ingredient often affects the chemical stability of the drug. Different crystallization conditions and storage conditions can lead to changes in the crystal structure of the compound, and sometimes the accompanying production of other forms of the crystalline form. In general, an amorphous drug product does not have a regular crystal structure, and often has other defects such as poor product stability, smaller particle size, difficult filtration, easy agglomeration, and poor flowability. Thus, it is necessary to improve various physical properties of the product. As such, it is necessary to study crystal structures having high purity and good chemical stability for a single compound.

### [Prior art documents]

### [Patent Documents]

(Patent Document 1) International Patent Application Publication No. WO 2008/007930
(Patent Document 2) International Patent Application Publication No. WO 2010/056022

### DISCLOSURE OF THE INVENTION

### TECHNICAL PROBLEM

An aspect of the present invention provides a stable co-crystal of a novel compound having an excellent agonistic activity, which is selective for a melanocortin receptor, in particular, melanocortin-4 receptor (MC4R), and a method for preparing the same.

Another aspect of the present invention provides a pharmaceutical composition comprising the stable co-crystal of the novel compound.

### TECHNICAL SOLUTION

According to an aspect of the present invention,
there is provided a co-crystal of a compound of the following formula 1 and vanillin, wherein the X-ray powder diffraction (XRPD) pattern has 5 or more characteristic peaks selected from among peaks with the following diffraction angles (2θ values) of: 5.85±0.2°, 7.23±0.2°, 9.08±0.2°, 11.18±0.2°, 14.47±0.2°, 14.81±0.2°, 15.43±0.2°, 16.35±0.2°, 16.80±0.2°, 17.48±0.2°, 18.05±0.2°, 18.85±0.2°, 19.00±0.2°, 19.97±0.2°, 20.29±0.2°, 21.30±0.2°, 21.65±0.2°, 22.48±0.2°, 22.80±0.2°, and 27.41±0.2°, 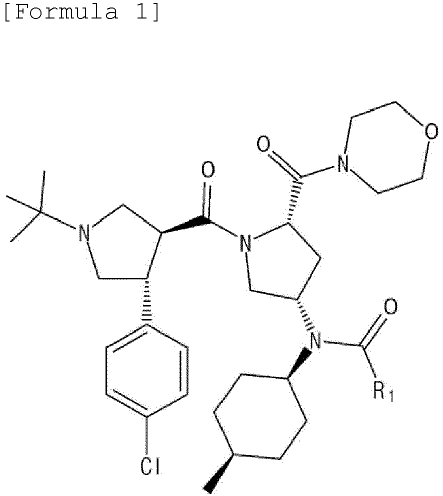 wherein
R₁ is a C₂-C₅ alkyl.

Since the compound of formula 1 can have an asymmetric carbon center and an asymmetric axis or an asymmetric plane, it can exist as cis or trans isomers, R or S isomers, racemates, diastereomer mixtures, and individual diastereomers, all of which are within the scope of the compound of formula 1.

In the present specification, unless otherwise specified for convenience, the compound of formula 1 is used to include all of the compound of formula 1, a pharmaceutically acceptable salt, an isomer, and a solvate thereof.

In one embodiment, according to the present invention, in formula 1, R₁ is a C₂ to C₅ alkyl. In another embodiment, according to the present invention, in formula 1, R₁ is a straight-chain or branched C₂ to C₅ alkyl, for example, ethyl, n-propyl, iso-propyl, n-butyl, iso-butyl, sec-butyl, or tert-butyl.

In another embodiment, according to the present invention, in formula 1, R₁ is a C₂ to C₄ alkyl. In another embodiment, according to the present invention, in formula 1, R₁ is a straight-chain or branched C₂ to C₄ alkyl, for example, ethyl, n-propyl, iso-propyl, n-butyl, sec-butyl, or tert-butyl. Specifically, R₁ may be iso-propyl.

In one embodiment, according to the present invention, the pharmaceutically acceptable salt of the compound of formula 1 includes, but is not limited to, acid-addition salts which are formed from inorganic acids, such as hydrochloric acid, sulfuric acid, nitric acid, phosphoric acid, hydrobromic acid, and hydroiodic acid; organic carboxylic acids, such as tartaric acid, formic acid, citric acid, acetic acid, trichloroacetic acid, trifluoroacetic acid, gluconic acid, benzoic acid, lactic acid, fumaric acid, and maleic acid; or sulfonic acids, such as methanesulfonic acid, benzenesulfonic acid, p-toluenesulfonic acid, or naphthalene-sulfonic acid.

In one embodiment, according to the present invention, the solvate may include a hydrate; and a solvate with an organic solvent, such as methanol, ethanol, 2-propanol, 1,2-propanediol, 1,3-propanediol, n-butanol, 1,4-butanediol, tert-butanol, acetic acid, acetone, methyl acetate, ethyl acetate, propyl acetate, n-butyl acetate, t-butyl acetate, isobutyl acetate, methylethylketone, 2-pentanone, tetrahydrofuran, acetonitrile, chloroform, toluene, and mixtures thereof.

In another embodiment, according to the present invention, the compound of formula 1, such as *N*-((3S,5S)-1-((3S,4R)-1-(*tert*-butyl)-4-(4-chlorophenyl)pyrrolidine-3-carbonyl)-5-(morpholine-4-carbonyl)pyrrolidin-3-yl)-*N-*((1s,4R)-4-methylcyclohexyl)isobutyramide may not form a co-crystal with L-arginine, choline hydroxide, meglumine, methylparaben, L-lysine, propyl gallate, sorbic acid, tromethamine, or urea. However, the present invention is not limited thereto.

In another embodiment, according to the present invention, the co-crystal of the compound of formula 1 and vanillin may have better chemical and/or physical stability and bioavailability than the compound of formula 1, the pharmaceutically acceptable salt thereof, the crystalline form thereof, or the solvate thereof. However, the present invention is not limited thereto.

In one embodiment, according to the present invention, the co-crystal of the compound of formula 1 and vanillin may have 5 or more, 7 or more, 9 or more, 10 or more, 13 or more, 15 or more, 17 or more, or 20 or more characteristic peaks selected from among 5.85±0.2°, 7.23±0.2°, 9.08±0.2°, 11.18±0.2°, 14.47±0.2°, 14.81±0.2°, 15.43±0.2°, 16.35±0.2°, 16.80±0.2°, 17.48±0.2°, 18.05±0.2°, 18.85±0.2°, 19.00±0.2°, 19.97±0.2°, 20.29±0.2°, 21.30±0.2°, 21.65±0.2°, 22.48±0.2°, 22.80±0.2°, and 27.41±0.2° in the X-ray powder diffraction pattern.

In another embodiment, according to the present invention, the co-crystal of the compound of formula 1 and vanillin may have characteristic peaks of 5.85±0.2°, 7.23±0.2°, 9.08±0.2°, 11.18±0.2°, 14.47±0.2°, 14.81±0.2°, 15.43±0.2°, 16.35±0.2°, 16.80±0.2°, 17.48±0.2°, 18.05±0.2°, 18.85±0.2°, 19.00±0.2°, 19.97±0.2°, 20.29±0.2°, 21.30±0.2°, 21.65±0.2°, 22.48±0.2°, 22.80±0.2°, and 27.41±0.2° in the X-ray powder diffraction pattern.

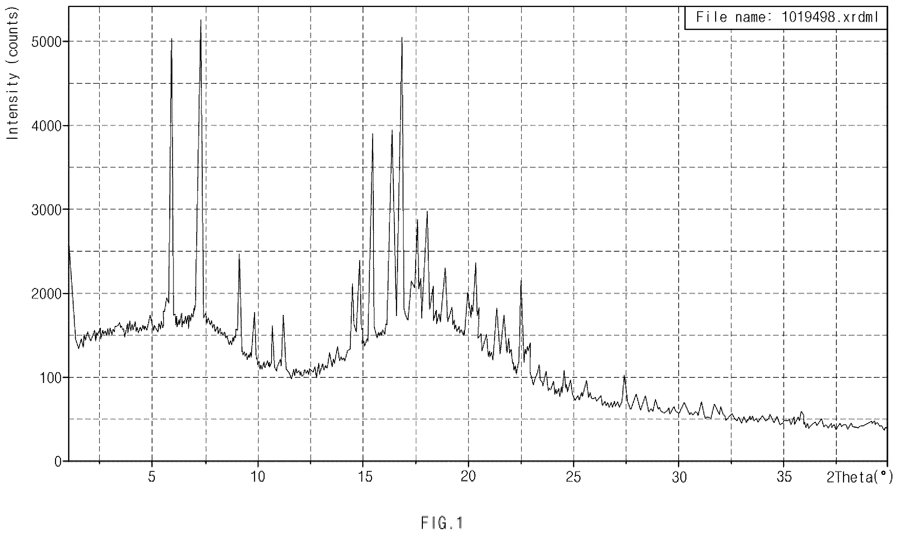

In one embodiment, according to the present invention, the co-crystal of the compound of formula 1 and vanillin may have the X-ray powder diffraction (XRPD) pattern shown in FIG. 1.

In one embodiment, according to the present invention, the co-crystal of the compound of formula 1 and vanillin may be an anhydrous substance. For example, this may be confirmed by the fact that no change was shown when the XRPD patterns were compared before and after drying the co-crystal of the compound of formula 1 and vanillin.

In one embodiment, according to the present invention, the co-crystal of the compound of formula 1 and vanillin may include the compound of formula 1 and vanillin in a molar ratio of 2:1 to 1:2, for example, 1:1 to 1:2, or 1:1.

The molar ratio of the compound of formula 1 and vanillin in the co-crystal of the compound of formula 1 and vanillin may be confirmed from, for example, ¹H NMR spectrum. However, a method for measuring the molar ratio is not limited thereto.

In another embodiment, according to the present invention, the co-crystal of the compound of formula 1 and vanillin may have the X-ray powder diffraction (XRPD) pattern shown in FIG. 1.

In another embodiment, according to the present invention, the co-crystal of the compound of formula 1 and vanillin, in the thermogravimetric analysis (TGA) profile, may have 10% or less, 8% or less, 6% or less, 5% or less, 4% or less, 3% or less, 2% or less, 1.5% or less or 1.4% of a weight loss in a temperature range of about 35°C to 180°C, and/or may be decomposed beyond about 260°C.

In another embodiment, according to the present invention, the co-crystal of the compound of formula 1 and vanillin, in the differential scanning calorimetry (DSC) profile, may have a peak showing at least one thermal behavior at 40°C to 130°C. For example, the co-crystal may have an endothermic peak at at least one temperature range of 40°C to 50°C, 75°C to 85°C, 90°C to 95°C, and 120°C to 130°C.

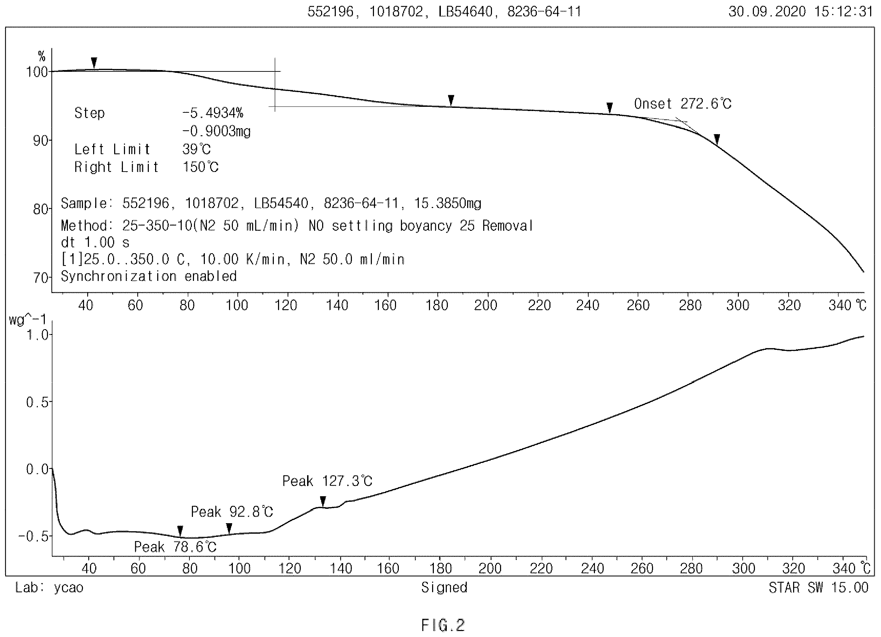

In another embodiment, according to the present invention, the co-crystal of the compound of formula 1 and vanillin may have the TGA (top) and/or DSC (bottom) profiles shown in FIG. 2.

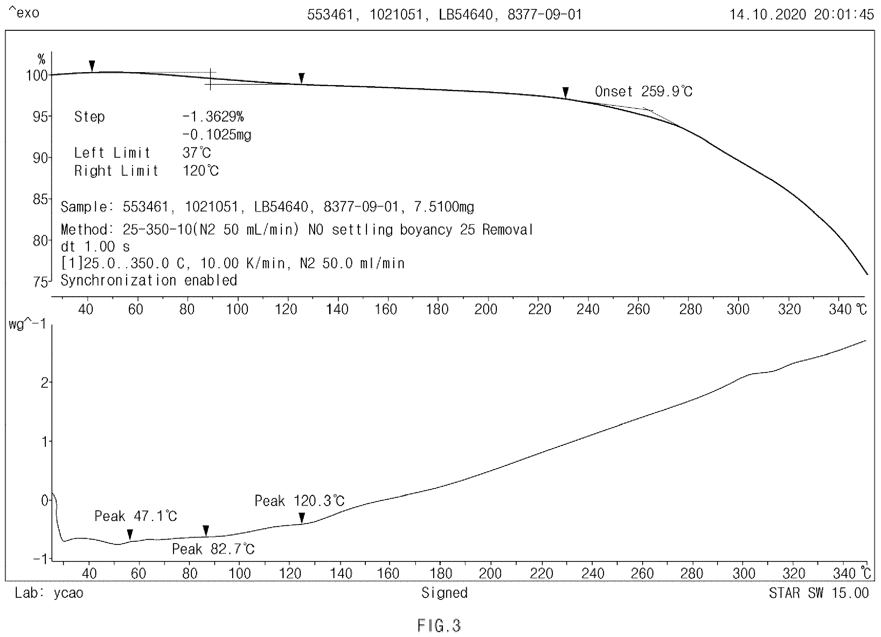

In another embodiment, according to the present invention, the co-crystal of the compound of formula 1 and vanillin may have the TGA (top) and/or DSC (bottom) profiles shown in FIG. 3.

In another embodiment, according to the present invention, the co-crystal of the compound of formula 1 and vanillin may be melted at about 70°C to 130°C, as determined by hot stage microscopy (HSM).

According to the DSC result and the HSM result, it may be inferred that the thermal behavior shown at about 70°C to 130°C on the DSC result resulted from the melting of the co-crystal based on the HSM result.

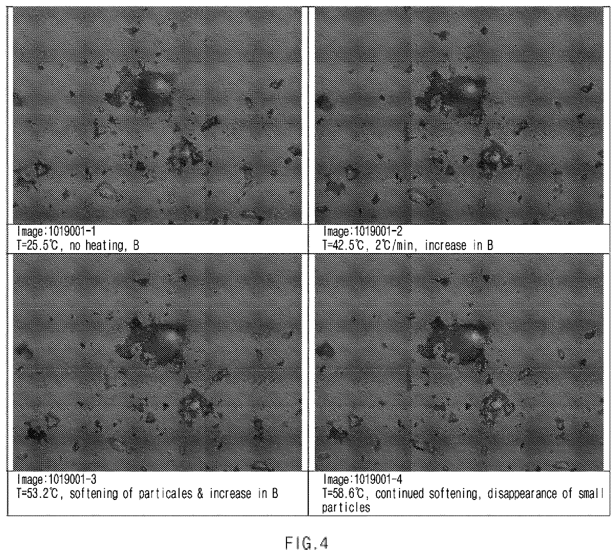

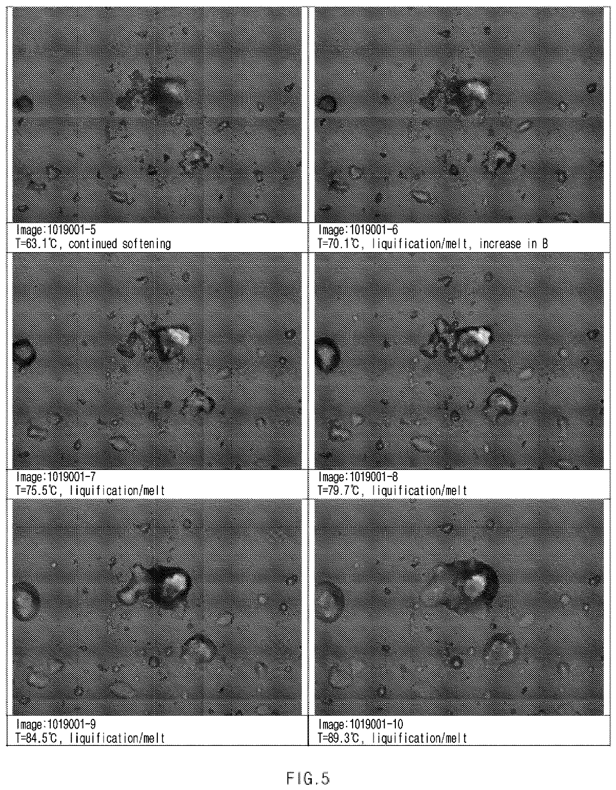

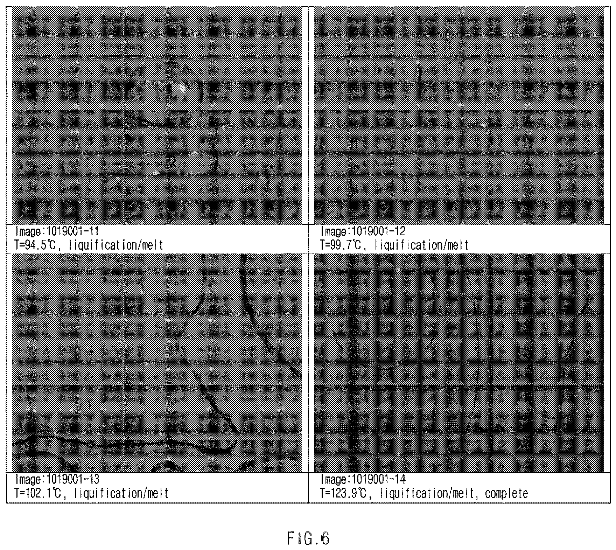

In another embodiment, according to the present invention, the co-crystal of the compound of formula 1 and vanillin may have the HSM photographs shown in FIGs. 4 to 6.

In another embodiment, according to the present invention, the co-crystal of the compound of formula 1 and vanillin may have a solubility of less than 1 mg/mL in an aqueous solution.

In another embodiment, according to the present invention, from the 1H NMR spectrum of the co-crystal of the compound of formula 1 and vanillin, it may be confirmed that the co-crystal includes the compound of formula 1 and vanillin in a molar ratio of 1:1.

In the present specification,
X-ray powder diffraction (XRPD) analysis shows the results obtained with PANalytical X' Pert Pro MPD system (Malvern Panalytical Ltd.).
Thermogravimetric analysis (TGA) and differential scanning calorimetry (DSC) analysis show the results obtained with TGA/DSC 3 (Mettler-Toledo AG).

Hot stage microscopy (HSM) shows the results obtained with Leica DM LP microscope using Linkman hot stage (model FTIR 600) with TMS 93 controller.

Solubility indicates the result of measurement based on the final volume of water added to a sample when it is visually confirmed that the sample is completely dissolved by adding water to the sample. The solubility of the actual sample may be higher than the measured solubility.

¹H NMR shows the result of measurement using Bruker Advance 600 MHz NMR spectrometer.

Herein, the temperature values may have an error of ±5°C.

The co-crystal of the compound of formula 1 and vanillin may have higher purity than the compound of formula 1, the pharmaceutically acceptable salt thereof, or crystalline forms or solvates thereof, and may be physically and chemically more stable.

In addition, the agonistic ability for the melanocortin-4 receptor and preventive or therapeutic effects on diseases, such as obesity, diabetes, inflammation, erectile dysfunction, or the like, of the co-crystal of the compound of formula 1 and vanillin, may be more excellent than those of known melanocortin-4 receptor agonists. However, the effects of the present invention are not limited thereto.

In another aspect, the present invention provides a method for preparing a co-crystal, comprising the steps of: preparing a mixed solution in which the compound of formula 1 and vanillin are mixed; and obtaining the co-crystal of the compound of formula 1 and vanillin from the mixed solution.

In one embodiment, according to the present invention, the compound of formula 1 for preparing the co-crystal may be a compound of formula 1, a salt thereof, an isomer thereof, or a solvate thereof.

The compound of formula 1 may be obtained by the preparation method described in the specification of application no. KR 10-2019-0141649 (filed on 7 November 2019) .

First, a mixed solution is prepared by mixing the compound of formula 1 and vanillin.

In one embodiment, according to the present invention, the molar ratio of the compound of formula 1 and vanillin in the mixed solution may be 2:1 to 1:2, for example, 1.5:1 to 1:1.5, 1:1 to 1:2, or 1:1.

The mixed solution may include, as a solvent, an organic solvent that is not particularly limited, so long as the organic solvent can dissolve the compound of formula 1 and vanillin.

The organic solvent may be, for example, a C₅ to C₁₀ aliphatic chain hydrocarbon solvent, a C₅ to C₁₀ alicyclic hydrocarbon solvent, a C₃ to C₂₀ ester solvent, a C₂ to C₂₀ ether solvent, a C₂ to C₂₀ organic nitrile solvent, or a mixed solvent thereof. Specifically, the organic solvent may include, but is not limited to, a solvent including at least one selected from the group consisting of hexane, heptane, cyclohexane, ethyl acetate, methyl tertiary-butyl ether and acetonitrile.

In one embodiment according to the present invention, the mixed solution may include acetonitrile, cyclohexane, and methyl tertiary-butyl ether as solvents.

In one embodiment, according to the present invention, the mixed solution may be prepared by mixing the compound of formula 1 and vanillin in an organic solvent (a first solvent), evaporating the first solvent, and then sequentially introducing at least one solvent different from the first solvent.

In another embodiment, according to the present invention, the mixed solution may be prepared by stirring the compound of formula 1 and vanillin in acetonitrile for a certain time, evaporating acetonitrile so that the mixture is gelated, adding cyclohexane and stirring for a certain time, evaporating cyclohexane, adding methyl tertiary-butyl ether and stirring for a certain time, evaporating methyl tertiary-butyl ether, and then adding hexane and stirring for a certain time.

In another embodiment, according to the present invention, the mixed solution may be prepared by stirring the compound of formula 1 and vanillin in ethyl acetate for a certain time, evaporating ethyl acetate, and then adding heptane and stirring for a certain time.

The mixing of the compound of formula 1 and vanillin may be performed at room temperature, for example, 20 to 40°C, and specifically, at a temperature of 25°C to 30°C.

Next, crystals are obtained from the mixed solution in which the compound of formula 1 and vanillin have been dissolved.

The crystals may be obtained, for example, by cooling the solution, by evaporating the solvent, by adding an antisolvent for supersaturation, or by using methods, such as slurry conversion, or the like.

In one embodiment, according to the present invention, the step of obtaining crystals may include evaporating the solvent from the mixed solution.

In one embodiment, according to the present invention, the co-crystal of the compound of formula 1 and vanillin may be obtained by evaporating the solvent from the mixed solution while stirring to produce a solid, and filtering the produced solid.

Stirring for evaporating the solvent may be performed, for example, at a temperature elevated from room temperature, for example, 30 to 80°C, and specifically, at a temperature of 40 to 60°C.

In one embodiment, according to the present invention, the stirring may be performed while gradually increasing the temperature of the mixed solution.

In another embodiment, according to the present invention, the co-crystal may be obtained by stirring the mixed solution at 50°C for a certain time and at 60°C for a certain time.

In another embodiment, according to the present invention, the co-crystal may be obtained by stirring the mixed solution at 40°C for a certain time, at 50°C for a certain time, and at 60°C for a certain time.

In one embodiment, according to the present invention, the co-crystal obtained as described above may be an anhydrous substance. That the co-crystal is obtained as an anhydrous substance can be confirmed by the fact that the characteristic peaks on the XRPD result graph before drying and the XRPD result graph after drying do not change after drying the co-crystal obtained as described above.

The co-crystal as obtained above may have higher purity than the compound of formula 1, the pharmaceutically acceptable salt thereof, or any crystalline forms of formula 1, and may be physically and chemically more stable. However, the effects of the present invention are not limited thereto.

In another aspect, the present invention provides a pharmaceutical composition comprising: (i) the co-crystal; and (ii) a pharmaceutically acceptable carrier.

The co-crystal, according to the present invention, exhibits excellent agonistic actions on melanocortin receptors, in particular, melanocortin-4 receptors (MC4R). Thus, the present invention can provide a pharmaceutical composition for agonizing melanocortin receptors, the composition containing the above-described co-crystal as an active ingredient. Specifically, the pharmaceutical composition may be a composition for agonizing the function of the melanocortin-4 receptor.

In addition, since the pharmaceutical composition can exhibit excellent effects of preventing or treating obesity, diabetes, inflammation, and erectile dysfunction, it may be a composition for preventing or treating obesity, diabetes, inflammation, or erectile dysfunction. However, the use of the present invention is not limited the diseases.

As used herein, "carrier" refers to a compound that facilitates the introduction of compounds into a cell or tissue.

When the co-crystal of the present invention is administered for clinical purposes, the total daily dose to be administered to a host in a single dose or in divided doses may be preferably in the range of 0.01 to 10 mg/kg body weight. However, the specific dose level for an individual patient may vary depending on the specific compound to be used, the patient's weight, sex, health status, diet, administration time of the drug, administration method, excretion rate, drug combination, the severity of the disease, or the like.

The co-crystal of the present invention may be administered by any route as desired. For example, the co-crystal of the present invention may be administered by injection or orally.

The pharmaceutical composition of the present invention may be in various oral dosage forms, such as tablets, pills, powders, capsules, granules, syrups, or emulsions, or parenteral dosage forms, such as injection preparations for intramuscular, intravenous, or subcutaneous administration.

Preparations for injection may be prepared, according to known techniques, using suitable dispersing agents, wetting agents, suspending agents, or excipients.

Excipients that can be used in the pharmaceutical preparation of the present invention include, but are not limited to, sweeteners, binders, solubilizers, solubilizing agents, wetting agents, emulsifiers, isotonic agents, adsorbents, disintegrants, antioxidants, preservatives, lubricants, fillers, fragrances, etc. For example, as excipients, lactose, dextrose, sucrose, mannitol, sorbitol, cellulose, glycine, silica, magnesium aluminum silicate, starch, gelatin, gum tragacanth, arginic acid, sodium alginate, methylcellulose, sodium carboxymethyl cellulose, water, ethanol, polyethylene glycol, polyvinyl pyrrolidone, sodium chloride, calcium chloride, orange essence, strawberry essence, vanilla flavor, etc. may be used.

When the pharmaceutical composition of the present invention is in an oral dosage form, examples of the carrier to be used may include, but are not limited to, cellulose, calcium silicate, corn starch, lactose, sucrose, dextrose, calcium phosphate, stearic acid, magnesium stearate, calcium stearate, gelatin, talc, etc.

When the pharmaceutical composition of the present invention is in an injectable preparation form, examples of the carrier may include, but are not limited to, water, saline, aqueous glucose solution, an aqueous sugar-like solution, alcohols, glycol, ethers, oils, fatty acids, fatty acid esters, glycerides, etc.

In another aspect, there is provided a co-crystal as described above for use in agonizing the functions of melanocortin receptors, in particular, melanocortin-4 receptors (MC4R).

In one embodiment, there is provided a co-crystal as described above for use in treating or preventing obesity, diabetes, inflammation, or erectile dysfunction.

In another aspect, there is provided a method for agonizing the function of melanocortin receptors, in particular, melanocortin-4 receptors (MC4R), the method comprising a step for administering to a subject the above-described co-crystal.

In another aspect, there is provided a method for treating obesity, diabetes, inflammation, or erectile dysfunction, the method comprising a step for administering to a subject the above-described co-crystal.

### ADVANTAGEOUS EFFECTS

The co-crystal, according to the present invention, exhibits excellent agonistic action on melanocortin receptors, in particular, melanocortin-4 receptors (MC4R), and thus can be usefully used for preventing or treating obesity, diabetes, inflammation, and erectile dysfunction.

The co-crystal, according to the present invention, exhibits an on-target effect on melanocortin-4 receptors, thereby exhibiting weight loss and diet reduction effects, without affecting anxiety and depression. In addition, it can be administered without any safety issues, such as side effects of human ether-a-go-go related gene (hERG) inhibition or mutagenesis.

In addition, the co-crystal, according to the present invention, has purity, yield, physical and chemical stability, which are more excellent than any compound of formula 1, the pharmaceutically acceptable salt thereof, or any crystalline forms of formula 1.

Specifically, the co-crystal may have superior moisture-absorbing property, solubility, storage stability, and production stability to the compound of formula 1, the pharmaceutically acceptable salt thereof, or any crystalline forms of formula 1.

### BRIEF DESCRIPTION OF THE DRAWINGS

FIG. 1 is a graph of the XRPD result of Example 1.
FIG. 2 is a graph of the TGA (top) and DSC (bottom) results of Example 1.
FIG. 3 is a graph of the TGA (top) and DSC (bottom) results of Example 2.
FIG. 4 is the HSM photograph of Example 1 (20x0.40 magnification).
FIG. 5 is the HSM photograph of Example 1 (20x0.40 magnification).
FIG. 6 is the HSM photograph of Example 1 (20x0.40 magnification).

### MODE FOR CARRYING OUT THE INVENTION

The Hereinafter, the present invention will be described in more detail through Preparation Examples and Examples. However, these Examples are merely illustrative of the present invention, and the scope of the present invention is not limited thereto.

### Preparation Example 1: Preparation of methyl (2S,4S)-4-(N-((1s,4R)-4-methylcyclohexyl)isobutyramido)pyrrolidine-2-carboxylate hydrochloride

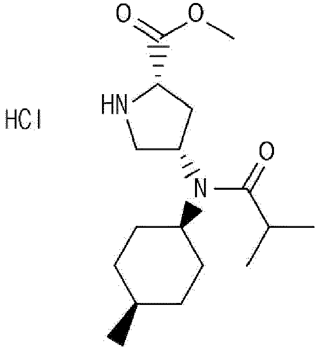

The title compound was obtained through the following steps A, B, C, D, and E.

### Step A: Preparation of 1-(tert-butyl) 2-methyl (2S,4S)-4-azidopyrrolidine-1,2-dicarboxylate

1-(tert-butyl) 2-methyl (2S,4R)-4-((methylsulfonyl)oxy)pyrrolidine-1,2-dicarboxylate (48.5 g, 150 mmol) was dissolved in N,N'-dimethylformamide (250 ml) under nitrogen, and sodium azide (19.5 g, 300 ml) was added. After stirring at 80°C for 16 hours, the reaction solvent was concentrated under reduced pressure, water was added, and extraction was performed twice with ethyl acetate. The organic layer was washed with an aqueous sodium chloride solution and water, dried over anhydrous magnesium sulfate, and filtered. The filtrate was concentrated under reduced pressure to obtain crude 1-(tert-butyl) 2-methyl (2S,4S)-4-azidopyrrolidine-1,2-dicarboxylate (39.59 g, 98%), which was used in the next step without purification.
MS [M+H] = 271 (M+1)
¹H NMR (400 MHz, CD3OD) δ 4.43-4.37 (m, 1H), 4.35-4.27 (br, 1H), 3.77 (s, 1.8H), 3.76 (s, 1.2H), 3.73-3.66 (m, 1H), 3.44-3.38 (m, 1H), 2.63-2.49 (m, 1H), 2.19-2.11 (m, 1H), 1.50 (s, 4.5H), 1.44 (s, 4.5H)

### Step B: Preparation of 1-(tert-butyl) 2-methyl (2S,4S)-4-aminopyrrolidine-1,2-dicarboxylate

1-(tert-butyl) 2-methyl (2S,4S)-4-azidopyrrolidine-1,2-dicarboxylate (24.59 g, 91.0 mmol) obtained in step A above was dissolved in tetrahydro (180 ml), and 1 M trimethylphosphine tetrahydrofuran solution (109.2 ml, 109.2 mmol) was slowly added at 0°C. The mixture was stirred at the same temperature for 1 hour and then at room temperature for 3 hours. After the reaction solvent was concentrated under reduced pressure, dichloromethane (100 ml) and water (150 ml) were added, and the mixture was stirred for about 30 minutes. The layers were separated and were extracted once more with dichloromethane, and the organic layer was dried over anhydrous magnesium sulfate and was filtered. The filtrate was concentrated under reduced pressure to obtain crude 1-(tert-butyl) 2-methyl (2S,4S)-4-aminopyrrolidine-1,2-dicarboxylate (20.62 g, 93%), which was used in the next step without purification.
MS [M+H] = 245 (M+1)
^{1H} NMR (400 MHz, CD3OD) δ 4.27 (m, 1H), 3.77 (s, 1.8H), 3.76 (s,1.2H), 3.75-3.67 (m, 1H), 3.50-3.42 (m, 1H), 3.22-3.17 (m, 1H), 2.58-2.47 (m,1H), 1.82-1.71 (m, 1H), 1.48 (s, 4.5H), 1.42 (s, 4.5H)

### Step C: Preparation of 1-(tert-butyl) 2-methyl (2S,4S)-4-(((1s,4R)-4-methylcyclohexyl)amino)pyrrolidine-1,2-dicarboxylate

1-(tert-butyl) 2-methyl (2S,4S)-4-aminopyrrolidine-1,2-dicarboxylate (20.62 g, 84.4 mmol) obtained in step B above was dissolved in dichloroethane (150 ml), and 4-methylcyclohexanone (9.5 ml, 101.3 mmol) was added. Sodium triacetoxyborohydride (26.8 g, 126.6 mmol) was added at 0°C, and the mixture was stirred at room temperature for 16 hours. The reaction solvent was concentrated under reduced pressure, water was added, and extraction was performed twice with ethyl acetate. The organic layer was washed with an aqueous sodium chloride solution, dried over anhydrous magnesium sulfate, and filtered. The filtrate was concentrated under reduced pressure and purified by column chromatography to obtain 1-(tert-butyl) 2-methyl (2S,4S)-4-(((1s,4R)-4-methylcyclohexyl)amino)pyrrolidine-1,2-dicarboxylate (22.9 g, 80%) .
MS [M+H] = 341 (M+1)
^{1H} NMR (400 MHz, CD3OD) δ 4.26 (m, 1H), 3.76 (s, 1.8H), 3.75 (s, 1.2H), 3.78-3.71 (m, 1H), 3.49-3.40 (m, 1H), 3.22-3.16 (m, 1H), 2.69-2.60(br, 1H), 2.58-2.46 (m, 1H), 1.87-1.77 (m, 1H), 1.73-1.63 (m, 1H), 1.62-1.35(m, 8H), 1.48 (s, 4.5H), 1.42 (s, 4.5H), 0.96 (d, 3H)

### Step D: Preparation of 1-(tert-butyl) 2-methyl (2S,4S)-4-(N-((1s,4R)-4-methylcyclohexyl)isobutyramido)pyrrolidine-1,2-dicarboxylate

1-(tert-butyl) 2-methyl (2S,4S)-4-(((1s,4R)-4-methylcyclohexyl)amino)pyrrolidine-1,2-dicarboxylate obtained in step C above (37.29 g, 109.5 mmol) was dissolved in dichloromethane (500 ml), triethyl amine (61.1 ml, 438.1 mmol) was added, and then isobutyryl chloride (11.7 ml, 219 mmol) was slowly added at 0°C. After the mixture was stirred at room temperature for 16 hours, the reaction solvent was concentrated under reduced pressure, an aqueous sodium hydrogen carbonate solution was added, and extraction was performed twice with ethyl acetate. The organic layer was washed with an aqueous sodium chloride solution and water, dried over anhydrous magnesium sulfate, and filtered. The filtrate was concentrated under reduced pressure, and purified by column chromatography to obtain 1-(tert-butyl) 2-methyl (2S,4S)-4-(N-((1s,4R)-4-methylcyclohexyl)isobutyramido)pyrrolidine-1,2-dicarboxylate (38.79 g, 86%).
MS [M+H] = 411 (M+1)
^{1H} NMR (400 MHz, CD3OD) δ 4.27 (m, 1H), 3.76 (s, 1.8H), 3.75 (s, 1.2H), 3.78-3.72 (m, 1H), 3.50-3.41 (m, 1H), 3.33-3.14 (m, 1H), 2.69-2.60 (m, 2H), 2.57-2.43 (m, 1H), 1.87-1.79 (m, 1H), 1.70-1.61 (m, 1H), 1.60-1.32 (m, 8H), 1.47 (s, 4.5H), 1.41 (s, 4.5H), 1.10 (dd, 6H), 0.99 (d, 3H)

### Step E: Preparation of methyl (2S,4S)-4-(N-((1s,4R)-4-methylcyclohexyl)isobutyramido)pyrrolidine-2-carboxylate hydrochloride

1-(tert-butyl) 2-methyl (2S,4S)-4-(N-((1s,4R)-4-methylcyclohexyl)isobutyramido)pyrrolidine-1,2-dicarboxylate (34.0 g, 82.8 mmol) obtained in step D above was dissolved in dichloromethane (200 ml), and a solution of 4 N hydrochloric acid in 1,4-dioxane solution (82.8 ml, 331.3 mmol) was added at 0°C. After the mixture was stirred at room temperature for 6 hours, the reaction solvent was concentrated under reduced pressure to obtain crude (28.7 g, 99%), which was used in the next step without purification.
MS[M+H] = 311 (M+1)

### Preparation Example 2: Preparation of (3S,4R)-1-(tert-butyl)-4-(4-chlorophenyl)pyrrolidine-3-carboxylic acid

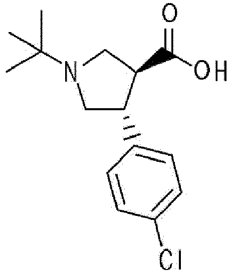

The title compound was obtained according to the method described in international patent publication no. WO 2004/092126.
MS[ M+H] = 282 (M+1)
^{1H} NMR (400 MHz, CD3OD) δ 7.43-7.33 (m, 4H), 3.90-3.69 (m, 3H), 3.59 (dd, J = 11.2, 10.0 Hz, 1H), 3.29 (dd, J = 11.2, 11.2 Hz, 1H), 3.18-3.09 (m, 1H), 1.44 (s, 9H)

### Preparation Example 3: Preparation of N-((3S,5S)-1-((3S,4R)-1-(tert-butyl)-4-(4-chlorophenyl)

### pyrrolidine-3-carbonyl)-5-(morpholine-4-carbonyl)pyrrolidin-3-yl)-N-((1s,4R)-4-methylcyclohexyl)isobutyramide (MC70)

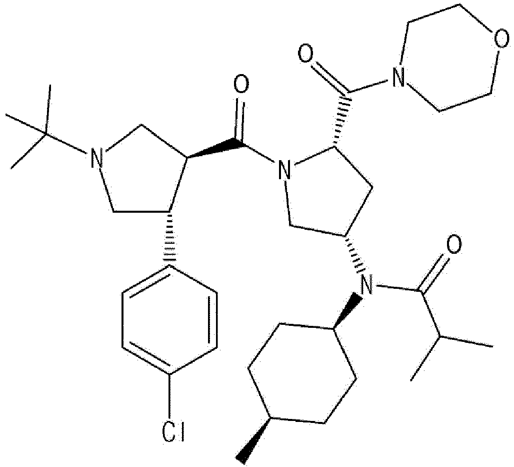

The title compound was obtained through the following steps A, B, and C.

### Step A: Preparation of methyl (2S,4S)-1-((3S,4R)-1-(tert-butyl)-4-(4-chlorophenyl)pyrrolidine-3-carbonyl)-4-(N-((1s,4R)-4-methylcyclohexyl)isobutyramido)pyrrolidine-2-carboxylate

Methyl (2S,4S)-4-(N-((1s,4R)-4-methylcyclohexyl)isobutyramido)pyrrolidine-2-carboxylate hydrochloride (28.7 g, 82.73 mmol) obtained in Preparation Example 1, (3S,4R)-1-(tert-butyl)-4-(4-chlorophenyl)pyrrolidine-3-carboxylic acid (24.5 g, 86.87 mmol) obtained in Preparation Example 2, 1-(3-dimethylaminopropyl)-3-ethylcarbodiimide hydrochloride (22.2 g, 115.83 mmol), and 1-hydroxybenzotriazole hydrate (15.7 g, 115.83 mmol) were dissolved in N,N'-dimethylformamide (400 ml), and N,N'-diisopropylethylamine (72.0 ml, 413.66 mmol) was added slowly. The mixture was stirred at room temperature for 16 hours, the reaction solvent was concentrated under reduced pressure, 0.5 N sodium hydroxide aqueous solution was added, and then, extraction was performed twice with ethyl acetate. The organic layer was washed twice with sodium chloride aqueous solution and water, dried over anhydrous magnesium sulfate, and filtered. The filtrate was concentrated under reduced pressure and purified by column chromatography to obtain methyl (2S,4S)-1-((3S,4R)-1-(tert-butyl)-4-(4-chlorophenyl)pyrrolidine-3-carbonyl)-4-(N-((1s,4R)-4-methylcyclohexyl)isobutyramido)pyrrolidine-2-carboxylate (41.19 g, 87%).
MS [M+H] = 575 (M+1)

### Step B: Preparation of (2S,4S)-1-((3S,4R)-1-(tert-butyl)-4-(4-chlorophenyl)pyrrolidine-3-carbonyl)-4-(N-((1s,4R)-4-methylcyclohexyl)isobutyramido)pyrrolidine-2-carboxylic acid

Methyl (2S,4S)-1-((3S,4R)-1-(tert-butyl)-4-(4-chlorophenyl)pyrrolidine-3-carbonyl)-4-(N-((1s,4R)-4-methylcyclohexyl)isobutyramido)pyrrolidine-2-carboxylate (39.4 g, 68.62 mmol) obtained in step A above was dissolved in methanol (450 ml), and then, 6 N sodium hydroxide aqueous solution (57.2 ml, 343.09 mmol) was added. The mixture was stirred at room temperature for 16 hours, the pH was adjusted to about 5 with 6 N aqueous hydrochloric acid solution, and then the reaction solution was concentrated under reduced pressure. The concentrate was dissolved in dichloromethane, and then the insoluble solid was filtered through a paper filter. The filtrate was concentrated under reduced pressure to obtain the crude title compound (38.4 g, 99%), which was used in the next step without purification.
MS [M+H] = 561 (M+1)

### Step C: Preparation of N-((3S,5S)-1-((3S,4R)-1-(tert-butyl)-4-(4-chlorophenyl)pyrrolidine-3-carbonyl)-5-(morpholine-4-carbonyl)pyrrolidin-3-yl)-N-((1s,4R)-4-methylcyclohexyl)isobutyramide

(2S,4S)-1-((3S,4R)-1-(tert-butyl)-4-(4-chlorophenyl)pyrrolidine-3-carbonyl)-4-(N-((1s,4R)-4-methylcyclohexyl)isobutyramido)pyrrolidine-2-carboxylic acid (38.4 g, 68.60 mmol) obtained in step B above, 1-(3-dimethylaminopropyl)-3-ethylcarbodiimide hydrochloride (18.4 g, 96.04 mmol), and 1-hydroxybenzotriazole hydrate (13.0 g, 96.04 mmol) were dissolved in N,N'-dimethylformamide (200 ml), and then morpholine (5.9 ml, 68.80 mmol) and N,N'-diisopropylethylamine (59.7 ml, 343.02 mmol) were sequentially and slowly added. The mixture was stirred at room temperature for 16 hours, the reaction solution was concentrated under reduced pressure, 0.5 N sodium hydroxide aqueous solution was added, and then extraction was performed twice with ethyl acetate. The organic layer was washed twice with sodium chloride aqueous solution and water, dried over anhydrous magnesium sulfate, and filtered. The filtrate was concentrated under reduced pressure and purified by column chromatography to obtain N-((3S,5S)-1-((3S,4R)-1-(tert-butyl)-4-(4-chlorophenyl)pyrrolidine-3-carbonyl)-5-(morpholine-4-carbonyl)pyrrolidin-3-yl)-N-((1s,4R)-4-methylcyclohexyl)isobutyramide (37.05 g, 86%, **MC70**).
MS [M+H] = 630 (M+1)

### Example 1: Preparation of co-crystal of N-((3S,5S)-1-((3S,4R)-1-(tert-butyl)-4-(4-chlorophenyl)pyrrolidine-3-carbonyl)-5-(morpholine-4-carbonyl)pyrrolidin-3-yl)-N-((1s,4R)-4-methylcyclohexyl)isobutyramide and vanillin

The compound (MC70) prepared above in Preparation Example 3 and vanillin in a molar ratio of 1:1 were added to acetonitrile, and the mixture was stirred at room temperature for 1 day. Acetonitrile was evaporated so that the mixture was gelated, cyclohexane was added, and the mixture was stirred at room temperature for 2 hours to prepare a gel-containing solution. Cyclohexane was evaporated so that the mixture was gelated again, and methyl tertiary-butyl ether was added to dissolve the gel. Methyl tertiary-butyl ether was evaporated again, then hexane was added to the gel, and the mixture was stirred at room temperature for 5 days to prepare a gel-containing solution. Next, the mixture was stirred at 50°C for three days and then at 60°C for three days to obtain the title co-crystal as a pink suspension.

### Example 2: Preparation of co-crystal of N-((3S,5S)-1-((3S,4R)-1-(tert-butyl)-4-(4-chlorophenyl)pyrrolidine-3-carbonyl)-5-(morpholine-4-carbonyl)pyrrolidin-3-yl)-N-((1s,4R)-4-methylcyclohexyl)isobutyramide and vanillin

The co-crystal according to Example 1 obtained above was dried under vacuum at 65°C for one day to obtain the title co-crystal.

### Example 3: Preparation of co-crystal of N-((3S,5S)-1-((3S,4R)-1-(tert-butyl)-4-(4-chlorophenyl)pyrrolidine-3-carbonyl)-5-(morpholine-4-carbonyl)pyrrolidin-3-yl)-N-((1s,4R)-4-methylcyclohexyl)isobutyramide and vanillin

The compound (MC70) prepared above in Preparation Example 3 and vanillin in a molar ratio of 1:1 were added to ethyl acetate, and the mixture was stirred at room temperature for 5 hours and at 40°C for 3 days. Ethyl acetate was evaporated, heptane was added, and then stirring was performed at room temperature for 5 hours to obtain a gel-containing solution. Next, the gel-containing solution was stirred at 40°C for 3 days and then 50°C for 3 days, and it was confirmed that some crystals were formed. In addition, stirring was carried out at 60°C for 9 days, and the title co-crystal was obtained as a dark red suspension.

### Comparative Example 1: Preparation of co-crystal of N-((3S,5S)-1-((3S,4R)-1-(tert-butyl)-4-(4-chlorophenyl)pyrrolidine-3-carbonyl)-5-(morpholine-4-carbonyl)pyrrolidin-3-yl)-N-((1s,4R)-4-methylcyclohexyl)isobutyramide and urea

The compound (MC70) prepared above in Preparation Example 3 and urea in a molar ratio of 1:1 were added to ethyl acetate, and the mixture was stirred at room temperature for one day to obtain a solid-containing solution. Then, even after the solid-containing solution was stirred at 50°C for 2 days and at 60°C for 14 days, it was confirmed that the title co-crystal was not formed.

### Experimental Example 1. XRPD assessment

Powder XRPD diffraction patterns were obtained with Panalytical Xpert Pro MPD diffractometer using an incident beam of Cu radiation. About 20 to 30 mg of the sample was compressed in a glass sample holder so that the sample had a flat surface, the generator of the apparatus was set to 45 kV (acceleration voltage) and 40 mA (filament emission), and then, the measurement was carried out in a reflection mode (not-spin). Bragg angles (2θ) in a range of 4 to 40° were measured with the conditions of step size of 0.026° and time per step of 51 seconds.

As can be seen from the spectrum shown in FIG. 1, it was confirmed that the co-crystal, according to Example 1, contained an amorphous material together with a crystalline substance to show a halo pattern. The specific values of the XRPD are shown in Table 1 below.

It was confirmed that the XRPD spectra of the co-crystal according to Example 2 and the co-crystal according to Example 3 also coincided with the XRPD spectrum of the co-crystal according to Example 1.

A comparison of the XRPD spectra between the co-crystal according to Example 1 and the co-crystal according to Example 2 showed that the shape of the co-crystal according to Example 1 did not change even when the co-crystal according to Example 1 was dried under vacuum condition at 65°C.

**[Table 1]**

| No. | 2θ | Relative intensity(I/I₀) |
|---|---|---|
| 1 | 5.8511 | 4667.76 |
| 2 | 5.98 | 357.15 |
| 3 | 7.2304 | 4765.99 |
| 4 | 9.0814 | 1435.57 |
| 5 | 9.81 | 473.22 |
| 6 | 10.675 | 606.16 |
| 7 | 11.173 | 796.87 |
| 8 | 13.4 | 201.09 |
| 9 | 13.817 | 292.63 |
| 10 | 14 | 422.68 |
| 11 | 14.458 | 1664.76 |
| 12 | 14.807 | 1992.44 |
| 13 | 15.4187 | 4730.83 |
| 14 | 16.345 | 4217.71 |
| 15 | 16.7975 | 6450.59 |
| 16 | 17.12 | 1102.36 |
| 17 | 17.325 | 1203.11 |
| 18 | 17.499 | 3244.84 |
| 19 | 17.688 | 1784.49 |
| 20 | 18.014 | 3596.96 |
| 21 | 18.276 | 962.06 |
| 22 | 18.49 | 1377.75 |
| 23 | 18.845 | 2128.24 |
| 24 | 19.136 | 763.77 |
| 25 | 19.47 | 1572.77 |
| 26 | 19.9 | 1531.73 |
| 27 | 20 | 100.41 |
| 28 | 20.12 | 948.92 |
| 29 | 20.283 | 2288.79 |
| 30 | 20.458 | 1224.78 |
| 31 | 20.6 | 534.48 |
| 32 | 20.83 | 1202.46 |
| 33 | 21.286 | 1850.4 |
| 34 | 21.64 | 1755.42 |
| 35 | 21.894 | 744.21 |
| 36 | 22.1 | 809.33 |
| 37 | 22.483 | 2487.3 |
| 38 | 22.724 | 612.24 |
| 39 | 22.868 | 1118.87 |
| 40 | 23.32 | 824.66 |
| 41 | 23.677 | 584.2 |
| 42 | 24.042 | 498.49 |
| 43 | 24.538 | 576.91 |
| 44 | 24.826 | 348.41 |
| 45 | 25.593 | 305.24 |
| 46 | 25.62 | 507.59 |
| 47 | 27.393 | 954.84 |
| 48 | 27.943 | 445.69 |
| 49 | 28.38 | 245.38 |
| 50 | 28.91 | 199.45 |
| 51 | 30.33 | 228.47 |
| 52 | 31.099 | 333.46 |
| 53 | 31.92 | 174.13 |
| 54 | 34.351 | 207.2 |
| 55 | 35.845 | 290.96 |
| 56 | 36.81 | 41.32 |

### Experimental Example 2. Thermogravimetric analysis (TGA) and differential scanning calorimetry (DSC)

TGA and DSC combo analysis was performed with Mettler-Toledo TGA/DSC 3+ analyzer. The sample was placed in an open aluminum pan, the pan was sealed, the lid was pierced, and then the pan was inserted into the TG furnace. Measurements were made by heating from 25°C to a maximum of 350°C at a rate of 10 K/min under nitrogen.

As can be seen in FIG. 2, according to the TGA analysis result, the co-crystal according to Example 1 had a weight loss of about 5.5 wt% in a temperature range of about 39°C to 180°C and was decomposed beyond about 270°C.

In addition, according to the DSC analysis result, for the co-crystal according to Example 1, several times of thermal behavior (peaks at 78.6°C, 92.8°C, 127.3°C) was observed at about 70°C to 130°C.

As can be seen in FIG. 3, according to the TGA analysis result, the co-crystal according to Example 2 had a weight loss of about 1.4 wt% in a temperature range of about 37°C to 120°C and was decomposed beyond about 260°C.

In addition, according to the DSC analysis result, for the co-crystal according to Example 2, endothermic peaks were observed at 47°C, 83°C, and 120°C.

From the above results, it was confirmed that the co-crystal according to Example 1 was an anhydrous substance.

### Experimental Example 3. Hot Stage Microscopy (HSM)

HSM was measured with Leica DM LP microscope using Linkman hot stage (model FTIR 600) with TMS 93 controller. Samples were observed with a lambda plate with crossed polarizers at either 10x0.22 or 20x0.40 magnification. Samples were placed on a coverslip, and another coverslip was placed over the sample. Then, the sample was visually observed as the stage was heated. In order to investigate outgassing, in some cases, a drop of mineral oil may be added to the sample. Images were captured using SPO Insight color digital camera with SPOT software v.4.5.9.

As can be seen in FIGs. 4 to 6, according to the HSM analysis result, it was confirmed that the co-crystal according to Example 1 started melting at 70°C and the melting was completed at 123.9°C.

Hence, it was inferred that several times of the thermal behavior on the DSC of Example 1 was due to the melting of the co-crystal.

### Experimental Example 4. Solubility assessment

Solubility was measured based on the final volume of water added to a sample when it was visually confirmed that the sample was completely dissolved by adding water to the sample.

According to the assessment result, the solubility of the co-crystal according to Example 2 was less than 1 mg/mL.

## Claims

1. A co-crystal of a compound of the following formula 1 and vanillin,
wherein the X-ray powder diffraction (XRPD) pattern has 5 or more characteristic peaks selected from among peaks with the following diffraction angles (2θ values) of: 5.85±0.2°, 7.23±0.2°, 9.08±0.2°, 11.18±0.2°, 14.47±0.2°, 14.8110.2°, 15.43±0.2°, 16.35±0.2°, 16.80±0.2°, 17.48±0.2°, 18.05±0.2°, 18.85±0.2°, 19.00±0.2°, 19.97±0.2°, 20.29±0.2°, 21.30±0.2°, 21.65±0.2°, 22.48±0.2°, 22.80±0.2°, and 27.41±0.2°, 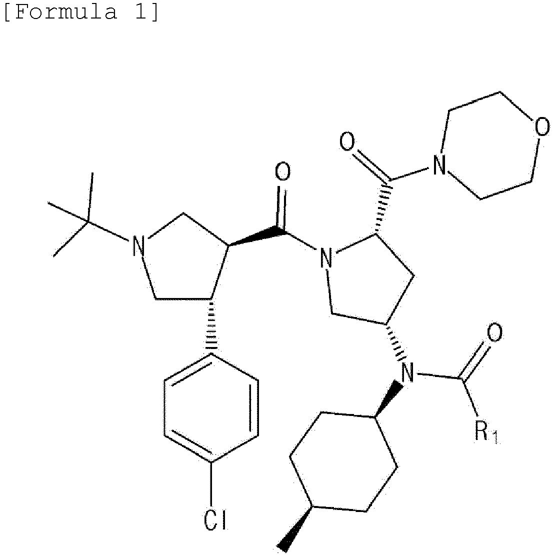
wherein
R₁ is a C₂-C₅ alkyl.

2. The co-crystal of claim 1, wherein R₁ is a C₂-C₄ alkyl.

3. The co-crystal of claim 2, wherein the compound of the formula 1 is *N*-((3S,5S)-1-((3S,4R)-1-(*tert*-butyl)-4-(4-chlorophenyl)pyrrolidine-3-carbonyl)-5-(morpholine-4-carbonyl)pyrrolidin-3-yl)-*N*-((1s,4R)-4-methylcyclohexyl)isobutyramide.

4. The co-crystal of claim 1, wherein the compound of the formula 1 and vanillin are included in a molar ratio of 2:1 to 1:2.

5. A method for preparing the co-crystal described in any one of claims 1 to 4, the method comprising the steps of:
preparing a mixed solution in which the compound of the formula 1 and vanillin are mixed; and
obtaining the co-crystal from the mixed solution.

6. The method for preparing the co-crystal of claim 5, wherein a molar ratio of the compound of the formula 1 and vanillin in the mixed solution is 2:1 to 1:2.

7. The method for preparing the co-crystal of claim 5, wherein the mixed solution includes a C₅ to C₁₀ aliphatic chain hydrocarbon solvent, a C₅ to C₁₀ alicyclic hydrocarbon solvent, a C₃ to C₂₀ ester solvent, a C₂ to C₂₀ ether solvent, a C₂ to C₂₀ organic nitrile solvent, or a mixed solvent thereof.

8. The method for preparing the co-crystal of claim 7, wherein the mixed solution includes a solvent including at least one selected from the group consisting of hexane, heptane, cyclohexane, ethyl acetate, methyl tertiary-butyl ether, and acetonitrile.

9. A pharmaceutical composition comprising the co-crystal according to any one of claims 1-4 and a pharmaceutically acceptable carrier.

10. A pharmaceutical composition for agonizing the function of a melanocortin-4 receptor, the composition comprising the co-crystal according to any one of claims 1-4 and a pharmaceutically acceptable carrier.

11. The pharmaceutical composition of claim 10, which is for preventing or treating obesity, diabetes, inflammation, or erectile dysfunction.
